# EUROPÄISCHE PATENTANMELDUNG

(11) **EP 1 929 937 A1**
(43) Veröffentlichungstag der Anmeldung: **11.06.2008**
(21) Anmeldenummer: 06025269.9
(22) Anmeldetag: 07.12.2006
(51) Int. Cl.: A61B 5/00

(54) **Vorrichtung und Verfahren zum Untersuchen von Körperflüssigkeiten**

(71) Anmelder: F. Hoffmann-La Roche AG, 4070 Basel (CH); Roche Diagnostics GmbH, 68305 Mannheim (DE)
(72) Erfinder: Haar, Hans-Peter, 69168 Wiesloch (DE); List, Hans, 64754 Hesseneck-Kailbach (DE)
(74) Vertreter: Pfiz, Thomas

(57) **Zusammenfassung**

Bei einer Vorrichtung zum Untersuchen von Körperflüssigkeiten mit einem längs einer Stechachse (48) in ein Körperteil (22) einstechbaren Stechelement (14), das ein Sammelvolumen (44) zum Sammeln von durch einen Einstich erhaltener Körperflüssigkeit (52) aufweist, und einem zum Nachweis eines Inhaltsstoffs der Körperflüssigkeit (52) ausgebildeten Aufnahmeelement (16), das mit Körperflüssigkeit (52) aus dem Sammelvolumen (44) beaufschlagbar ist, sind erfindungsgemäß das Stechelement (14) und das Aufnahmeelement (16) in der Stechachse (48) relativ zueinander beweglich angeordnet, so dass das Aufnahmeelement (16) während einer auf den Einstich folgenden Transferphase in Stechrichtung in das Sammelvolumen (44) eintaucht.

## Beschreibung

Die Erfindung betrifft eine Vorrichtung zum Untersuchen von Körperflüssigkeiten, insbesondere für Blutzuckertests, mit einem längs einer Stechachse in ein Körperteil einstechbaren Stechelement, das ein Sammelvolumen zum Sammeln von durch einen Einstich erhaltener Körperflüssigkeit aufweist, und einem zum Nachweis eines Inhaltsstoffs der' Körperflüssigkeit ausgebildeten Aufnahmeelement, das mit Körperflüssigkeit aus dem Sammelvolumen beaufschlagbar ist. Die Erfindung betrifft weiter ein entsprechendes Untersuchungsverfahren und ein Verfahren zur Sterilisierung einer solchen als Einwegteil ausgebildeten Vorrichtung.

In einer früheren Anmeldung PCT/EP2006/009945 der Anmelder ist ein Testelement zur Untersuchung von Körperflüssigkeit für Analysezwecke, speziell zur Blutglucose-Konzentrationsbestimmung beschrieben. Daraus geht ein Stechelement mit einem Sammelbereich für Körperflüssigkeit hervor, wobei der Sammelbereich durch eine in Stechrichtung langgestreckte Sammelausnehmung gebildet ist und ein fest integrierter Lichtleiter mit seinem distalen Ende in einer proximalen Messzone der Sammelausnehmung angeordnet ist. Damit lässt sich über einen mikrofluidischen Flüssigkeitstransfer innerhalb einer Stechstruktur ein optischer Nachweis eines Analyten verwirklichen.

Ausgehend hiervon liegt der Erfindung die Aufgabe zugrunde, die im Stand der Technik bekannten Systeme weiter zu entwickeln und eine Vorrichtung und ein Verfahren der eingangs angegebenen Art im Sinne einer zuverlässigen Probenverarbeitung zu optimieren, wobei unter Reduzierung des Einstichschmerzes möglichst geringe Probenmengen angestrebt werden und ein Erfindungsziel auch in einer vereinfachten Herstellung besteht.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, ein überschüssiges Volumen für einen kapillaren Probentransport zu vermeiden und stattdessen durch eine geeignete Relativbewegung das Aufnahmeelement in direkten Probenkontakt zu bringen. Dementsprechend wird erfindungsgemäß vorgeschlagen, dass das Stechelement und das Aufnahmeelement in der Stechachse relativ zueinander beweglich angeordnet sind, so dass das Aufnahmeelement während einer auf den Einstich folgenden Transferphase in Stechrichtung in das Sammelvolumen eintaucht. Dadurch wird eine hinreichende Flüssigkeitsbenetzung des Aufnahmeelements auch ohne Kapillartransport der Flüssigkeit möglich, wobei durch das gezielte Eintauchen Funktionsstörungen weitgehend vermieden werden. Somit ist es möglich, mit kleinsten Probenmengen in einer miniaturisierten Bauform weitgehend automatisch einen vollständigen Messablauf durchzuführen. Das Aufnahmeelement kann dabei lediglich einen weiteren Probentransport vermitteln oder aber direkt als sensorisches Element wirken.

In bevorzugter Ausgestaltung ist der Abstand zwischen dem Stechorgan des Stechelements und dem Aufnahmeelement in der Transferphase kleiner ist als in einer vorausgehenden Stechphase. In der Stechphase wird somit ein Körperkontakt des Aufnahmeelements vermieden, wäh- ' rend durch die Abstandsverringerung die Probenmenge entsprechend reduziert werden kann.

Vorteilhafterweise ist das Aufnahmeelement durch eine Rückziehbewegung eines mit dem Stechelement gekoppelten Stechantriebs in das Sammelvolumen einführbar. Alternativ ist es auch möglich, dass das Aufnahmeelement mittels eines Vorschubantriebs in das Sammelvolumen hinein bewegbar ist.

Um gerätefeste Antriebseinheiten nutzen zu können, ist es vorteilhaft, wenn das Stechelement und/oder das Aufnahmeelement an einem proximalen Ende eine Andockstruktur für eine lösbare Antriebskopplung aufweisen.

Für einen vereinfachten Messablauf ist es vorteilhaft, wenn das Stechelement in einer vorzugsweise durch einen zugeordneten Köcher gebildeten Linearführung gelagert ist.

Eine baulich vorteilhafte Ausführung sieht vor, dass das Aufnahmeelement in eine zu dem Sammelvolumen hin führende Ausnehmung des Stechelements eingreift.

Besonders bevorzugt sind das Stechelement und das Aufnahmeelement zusammengehörig als Einwegteil ausgebildet. Auf diese Weise lassen sich Messungen mit hohem Benutzerkomfort durchführen. Eine weitere Verbesserung in dieser Hinsicht kann durch ein zur Aufnahme einer Vielzahl von Stechelementen und zugehörigen Aufnahmeelementen ausgebildetes Magazin, insbesondere Scheiben-, Trommel- oder Stapelmagazin erreicht werden.

Für eine weiter erhöhte Messintegration ist es vorteilhaft, wenn das Aufnahmeelement ein Messorgan für eine optische oder elektrochemische Messung in der aufgenommenen Körperflüssigkeit aufweist. Hierbei ist es bevorzugt, wenn das Messorgan mit einem unter Flüssigkeitskontakt mit dem Inhaltsstoff der Körperflüssigkeit reagierenden Testreagenz beschichtet ist.

Für eine Signalübermittlung an eine Geräteeinheit ist es von Vorteil, wenn das Aufnahmeelement über einen in dem Stechelement geführten Lichtleiter mit einer Messeinheit verbindbar oder verbunden ist.

Zur optimierten Flüssigkeitsaufnahme ist es günstig, wenn das Sammelvolumen durch einen beidseitig offenen Schlitz oder eine einseitig offene Rinne des Stechelements gebildet ist.

Ein besonderer Aspekt der Erfindung liegt auch darin, dass das Sammelvolumen weniger als 50 nl, bevorzugt weniger als 10 nl Körperflüssigkeit aufnimmt. Auf diese Weise kann eine weitere Schmerzreduktion bei der Probengewinnung und damit eine erhöhte Benutzerakzeptanz erreicht werden. Durch die besondere Probenaufnahme genügt es, wenn das Aufnahmeelement an einer distalen Frontseite mit einem Aliquot der Körperflüssigkeit aus dem Sammelvolumen beaufschlagt ist, wobei das Aliquot weniger als 5 nl, bevorzugt weniger als 1 nl beträgt.

Gegenstand der Erfindung ist auch ein Stechgerät zum Einsatz mindestens einer als Einwegteil ausgebildeten erfindungsgemäßen Vorrichtung, mit einem durch einen Benutzer auslösbaren Stechantrieb, wobei das Einwegteil in ungebrauchtem Zustand in einer ersten Position und nach dem Einstich in gebrauchtem Zustand in einer davon entfernten zweiten Position im Geräteinneren gelagert ist. Dadurch kann die Bereitstellung und Entsorgung ohne Benutzermanipulation gewährleistet werden.

In verfahrensmäßiger Hinsicht wird die eingangs genannte Aufgabe dadurch gelöst, dass während einer auf den Einstich folgenden Transferphase das Stechelement und das Aufnahmeelement in der Stechachse relativ zueinander bewegt werden, so dass das Aufnahmeelement in Stechrichtung in das Sammelvolumen eintaucht. Auch hier ergeben sich die bereits vorstehend aufgeführten Vorteile.

In vorteilhafter Ausgestaltung wird der Abstand zwischen dem Aufnahmeelement und einem distalen Stechorgan des Stechelements in der Transferphase verringert, wobei das Aufnahmeelement bei dem Einstich mit dem Stechelement mitbewegt wird, oder aber das Stechelement in einer hin- und hergehenden Stechbewegung angetrieben wird, während das Aufnahmeelement ortsfest gehalten wird.

Vorteilhafterweise ist das Stechelement in der Transferphase außerhalb des die Körperflüssigkeit enthaltenden Körperteils befindlich, wobei es weiter von Vorteil ist, wenn über das Aufnahmeelement als Messorgan eine optische oder elektrochemische Messung in der aufgenommenen Körperflüssigkeit durchgeführt wird.

Ein weiterer Erfindungsaspekt besteht in einem Verfahren zur Sterilisierung einer als Einwegteil ausgebildeten erfindungsgemäßen Vorrichtung, bei welchem das mit einem Testreagenz beschichtete Aufnahmeelement im Abstand von einem distalen Stechorgan des Stechelements gehalten wird und das Stechorgan vorzugsweise mit einem Elektronenstrahl bestrahlt wird. Hierbei ist es möglich, dass die Strahlung, insbesondere der Elektronenstrahl so auf das Stechorgan fokussiert wird und/oder der Abstand zwischen Aufnahmeelement und Stechorgan so gewählt wird, dass das Testreagenz durch die Strahlung nicht geschädigt wird.

Im Folgenden wird die Erfindung anhand der,in der Zeichnung schematisch dargestellten Ausführungsbeispiele näher erläutert. Es zeigen
- Fig. 1a: ein Gerät zur Blutzuckermessung mit einem disposiblen Stech- und Nachweiselement in einem Blockschaltbild;
- Fig. 1b und c: das Stech- und Nachweiselement nach Fig. 1a in einer Stech- und Nachweisstellung;
- Fig. 2: ein Scheibenmagazin mit einem aktiven Stech- und Nachweiselement in verschiedenen Stellungen in der Draufsicht;
- Fig. 3: eine weitere Ausführungsform eines Stech- und Nachweiselements in zwei Stellungen in perspektivischer Darstellung;
- Fig. 4: noch eine weitere Ausführungsform eines Stech- und Nachweiselements in verschiedenen Stellungen im Axialschnitt.

Die in der Zeichnung dargestellte diagnostische Messanordnung umfasst ein Analysegerät 10 für Blutzuckermessungen und mindestens ein als Verbrauchsteil für den Einmalgebrauch darin einsetzbares Testelement 12, welches ein Stechelement 14 und ein Aufnahme- bzw. Nachweiselement 16 aufweist.

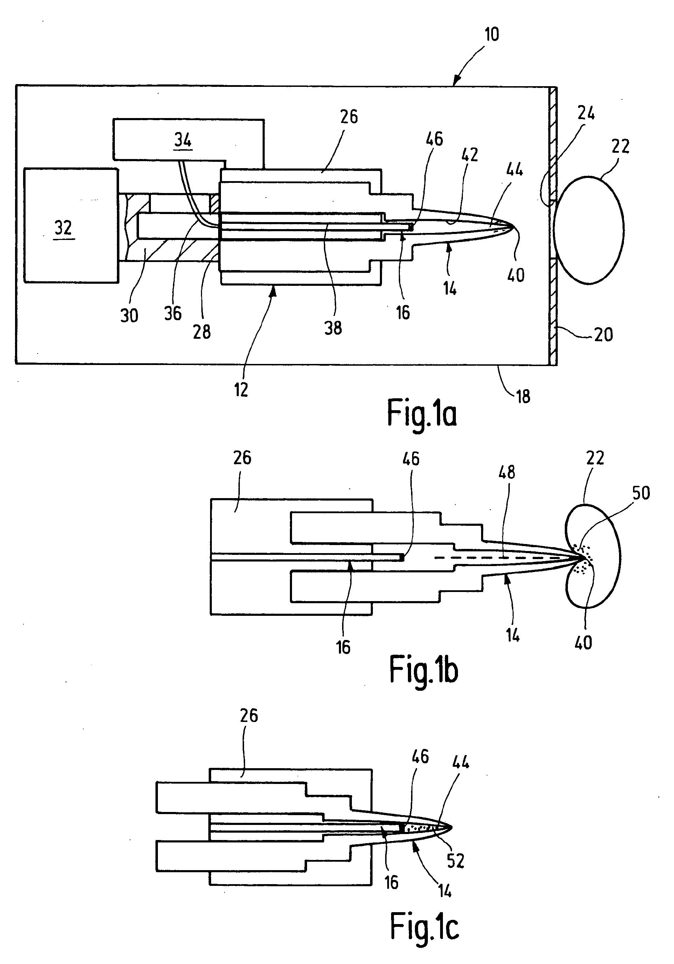

Wie aus Fig. 1a ersichtlich, besitzt das Analysegerät 10 ein Gerätegehäuse 18 mit einer Auflage 20 beispielsweise für einen Finger 22 im Bereich einer Durchstechöffnung 24. Das Stechelement 14 ist innerhalb des Gehäuses 18 in einer Linearführung 26 gelagert, um eine hin- und hergehende Stechbewegung gegen den aufgelegten Finger 22'auszuführen. Zu diesem Zweck ist das Stechelement 14 über eine proximale Andockstruktur 28 mit dem Stößel 30 eines Stechantriebs 32 lösbar verbindbar. Für einen optischen Glucosenachweise in einer mittels des Stechelements 14 erhaltenen Blutprobe ist eine geräteinterne Nachweiseinheit 34 vorgesehen, die über eine flexible Leitung 36 an einen Lichtleiter 38 des Nachweiselements 16 ankoppelbar ist.

Das Stechelement 14 ist als Flachteil beispielsweise aus Edelstahlblech gebildet und besitzt eine distale Spitze 40 als Stechorgan, in deren rückseitigem Bereich sich ein durch einen beidseitig offenen Schlitz 42 begrenztes Sammelvolumen 44 befindet.

Das Nachweiselement 16 greift mit seinem Lichtleiter in den Schlitz 42 ein, wobei eine mit einem Testreagenz 46 beschichtete Frontfläche des Lichtleiters 38 frontal zu dem Sammelvolumen 44 hin ausgerichtet ist. Das Testreagenz 46 verfärbt sich bei Beaufschlagung mit Blutflüssigkeit durch darin enthaltene Glucose, so dass aufgrund der Farbänderung eine optische Detektion über die Lichtleiter 38, 36 mittels der Nachweiseinheit 34 möglich ist. Das Messergebnis lässt sich für den Benutzer anzeigen, um so vor Ort eine Selbstuntersuchung zu ermöglichen.

Wie aus den Fig. 1a,b,c ersichtlich, ist das Stechelement 14 in der Führung 26 relativ zu dem gerätefest gehaltenen Nachweiselement 16 linear beweglich angeordnet. Das Stechelement 14 ist also entlang einer Stechachse 48 hin- und herbeweglich, so dass ein Einstich in das Körperteil 22 ausführbar ist und die dabei gewonnene Blutprobe auf das Testreagenz 46 übertragbar ist. In der Ausgangsstellung nach Fig. 1a ist die Stechbewegung durch den Benutzer nach Auflegen des Fingers 22 auslösbar. In der darauf folgenden Stechphase dringt das Stechorgan 40 bei geeigneter Einstellung der Stechtiefe in eine blutgebende Hautschicht 50 ein. Während der Rückziehbewegung, die generell langsamer erfolgt als die schnelle Vorwärtsbewegung beim Einstich, wird Körperflüssigkeit 52 (Blut, ggf. auch Gewebeflüssigkeit) in dem Sammelvolumen 44 aufgenommen. Hierbei genügt eine mikroskopische Blutmenge im Bereich von 10 Nanoliter oder weniger. In der abschließenden Transferphase wird durch die Rückbewegung des Stechelements 14 ein Teil der gesammelten Körperflüssigkeit 52 auf das Nachweiselement 16 übertragen, wobei die Reagenzschicht 46 als sensorisches Element proximal in das Sammelvolumen 44 eintaucht. Aufgrund der Abstandsverringerung zwischen dem Sensorelement (Testreagenz 46) und dem Stechorgan 40 bzw. dem Sammelvolumen 44 müssen somit keine überschüssigen Flüssigkeitsvolumina für einen Kapillartransport der Körperflüssigkeit aufgenommen werden. Für die eigentliche Nachweisreaktion an der Lichtleiterspitze genügt ein äußerst geringes Teilvolumen von etwa 1 Nanoliter.

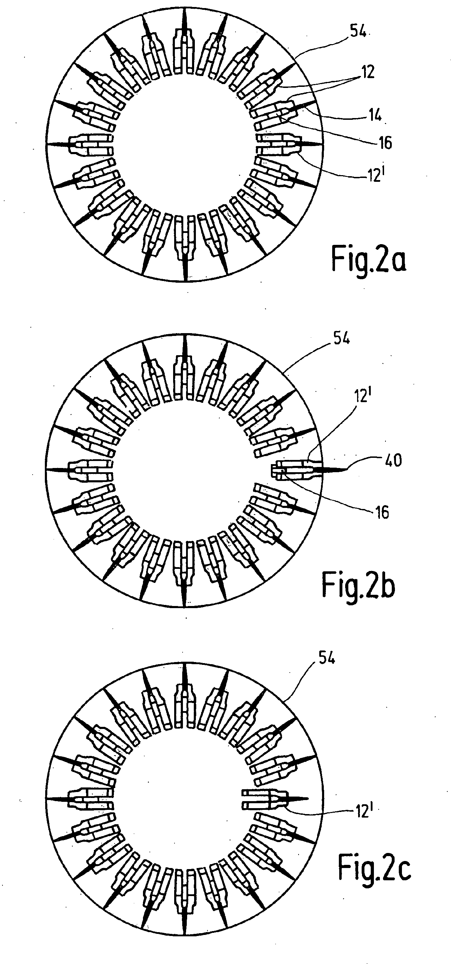

Fig. 2 veranschaulicht den möglichen Einsatz einer Vielzahl von Testelementen 12 in einem Scheibenmagazin 54. Durch Drehung eines solchen Scheibenmagazins 54 in einem Gerät 10 können die Testelemente sequentiell in eine aktive Stellung (Testelement 12') gebracht werden. Gemäß Fig. 2b erfolgt dann der Einstich unter Abstandsvergrößerung zwischen dem Stechorgan 40 und dem Nachweiselement 16, während in der Transferphase nach Fig. 2c durch Abstandsverringerung ein Flüssigkeitstransfer erreicht wird. Anschließend kann das gebrauchte Testelement durch Weiterschalten des Magazins 54 entsorgt werden.

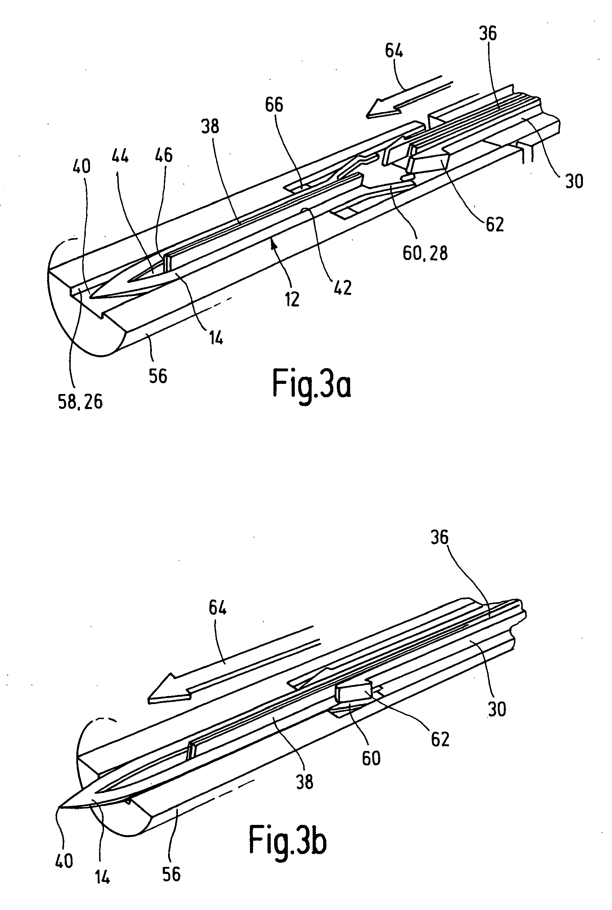

Fig. 3 zeigt eine Ausführungsform eines Testelements 12 in einem zugeordneten zylindrischen Köcher 56, welcher einen Teil des Einwegartikels bildet und mit einer Zentralausnehmung 58 eine Führung 26 für das U-förmig längsgeschlitzte Stechelement 14 bildet. Ein in dem Schlitz 42 des Stechelements 14 angeordneter Lichtleitstab 38 ist ebenfalls als Teil des Verbrauchsartikels fest mit dem Köcher 56 verbunden. Der Lichtleitstab 38 ist frontseitig mit dem Testreagenz 46 beschichtet und rückseitig an den geräteseitig mit der Nachweiseinheit 34 gekoppelten Lichtleiter 36 andockbar. Zu diesem Zweck ist als Andockstruktur 28 an dem Stechelement 14 eine zangenartige Klauenkupplung 60 vorgesehen, die bei der Vorschubbewegung (Pfeil 64) mit dem Kopfstück 62 des Antriebsstößels 30 selbsttätig formschlüssig in Eingriff kommt. Hierfür sind an dem Köcher 60 innenseitig Anlaufschrägen 66 ausgebildet, welche die elastisch aufspreizenden Klauen 60 schließen, so dass auch eine Rückholbewegung nach der Blutaufnahme möglich ist. In dieser Ausführung ist es möglich, dass der Köcher 56 durch einen gesonderten Vorschubantrieb bis an den Finger 22 herangefahren wird, wobei die freie Stirnseite als Referenzfläche für die Stechtiefe gegen die Haut drückt und diese stabilisiert.

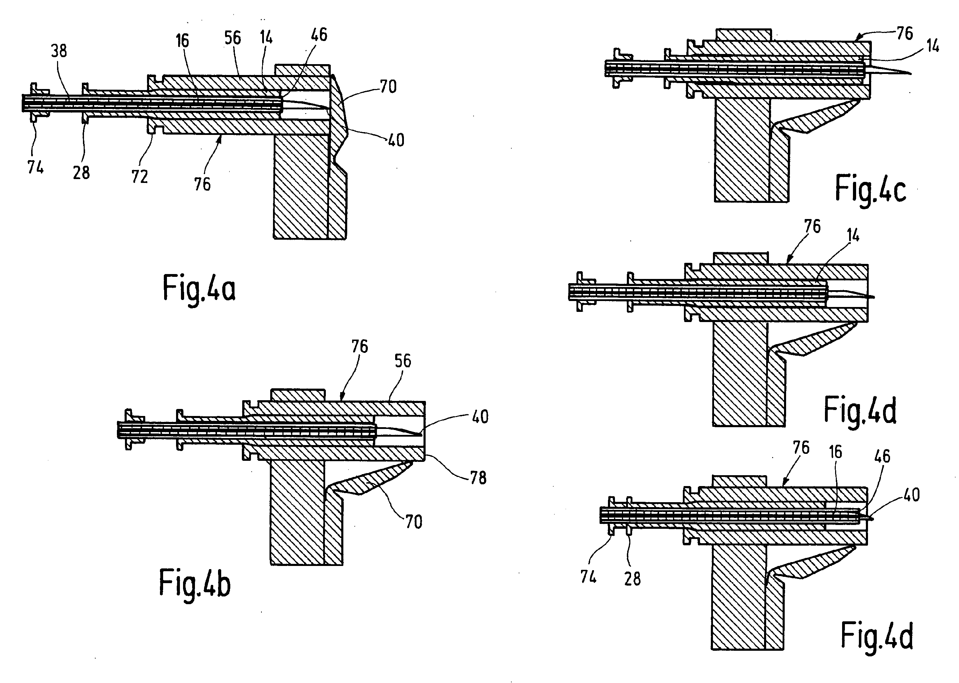

Bei der in Fig. 4 gezeigten Ausführungsform sind ebenfalls gleiche Teile mit gleichen Bezugszeichen wie vor- ' stehend beschrieben versehen. In der Ausgangsposition Fig. 4a ist der Köcher 56 an seiner Frontöffnung durch eine Siegelklappe 70 steril verschlossen. Das Testreagenz 46 bleibt somit auch gegen Umwelteinflüsse und insbesondere Feuchte geschützt. Der Köcher 56, das Stechelement 14 und das Nachweiselement 16 sind jeweils am proximalen Ende mit einer Andockstruktur 72, 28, 74 für eine gesonderte Antriebskopplung versehen. In der gezeigten Ablaufsequenz wird zunächst über einen Vor- - schub des gesamten Einwegteils 76 die Siegelklappe 70 geöffnet und ein stirnseitiger Hautkontakt hergestellt (Fig. 4b). Beim Aufstoßen der Klappe 70 durch die Stirnseite 78 des Köchers 56 wird die empfindliche Stechspitze 40 geschont. Sodann folgen der Stechvorschub des Stechelements 14 bis zur maximalen Eindringtiefe (Fig. 4c) und die anschließende Rückholbewegung ' (Fig. 4d). In der dadurch definierten Stechachse erfolgt dann eine separate Vorschubbewegung des Nachweiselements 16, so dass das stirnseitige Testreagenz 46 näher zum Stechorgan 40 gelangt und dabei in das Sammelvolumen 44 eintaucht (Fig. 4e).

Beim Einstich gemäß Fig. 4c schützt der relativ große Abstand von Nachweiselement 16 und Stechorgan 40 den Probanden vor einer Kontamination mit dem Testreagenz 46. Darüber hinaus erlaubt dieser Abstand auch eine vereinfachte Sterilisierung der Stechspitze 40 im Zuge der Herstellung. Dabei kann ein Elektronenstrahl auf die Spitze 40 fokussiert werden, ohne dass die Strahlung das im geeigneten Abstand befindliche Testreagenz 46 schädigen bzw. funktionsuntauglich machen würde. Insbesondere kann hierbei auf gesonderte Barrieren zwischen Testreagenz und Stechspitze verzichtet werden.

## Patentansprüche

1. Vorrichtung zum Untersuchen von Körperflüssigkeiten, insbesondere für Blutzuckertests, mit einem längs einer Stechachse (48) in ein Körperteil (22) einstechbaren Stechelement (14), das ein Sammelvolumen (44) zum Sammeln von durch einen Einstich erhaltener Körperflüssigkeit (52) aufweist, und einem zum Nachweis eines Inhaltsstoffs der Körperflüssigkeit (52) ausgebildeten Aufnahmeelement (16), das mit Körperflüssigkeit (52) aus dem Sammelvolumen (44) beaufschlagbar ist, **dadurch gekennzeichnet, dass** das Stechelement (14) und das Aufnahmeelement (16) in der Stechachse (48) relativ zueinander beweglich angeordnet sind, so dass das Aufnahmeelement (16) während einer auf den Einstich folgenden Transferphase in Stechrichtung in das Sammelvolumen (44) eintaucht.

2. Vorrichtung nach Anspruch 1, **dadurch gekennzeichnet, dass** das Stechelement (14) ein distales Stechorgan (40) aufweist, und dass der Abstand zwischen dem Stechorgan (40) und dem Aufnahmeelement (16) in der Transferphase kleiner ist als in einer vorausgehenden Stechphase.

3. Vorrichtung nach Anspruch 1 oder 2, **dadurch gekennzeichnet, dass** das Aufnahmeelement (16) durch eine Rückziehbewegung eines mit dem Stechelement (14) gekoppelten Stechantriebs (32) in das Sammelvolumen (44) einführbar ist.

4. Vorrichtung nach Anspruch 1 oder 2, **dadurch gekennzeichnet, dass** das Aufnahmeelement (16) mittels eines Vorschubantriebs (74) in das Sammelvolumen (44) hinein bewegbar ist.

5. Vorrichtung nach einem der Ansprüche 1 bis 4, **dadurch gekennzeichnet, dass** das Stechelement (14) und/oder das Aufnahmeelement (16) an einem proximalen Ende eine Andockstruktur (28;74) für eine lösbare Antriebskopplung aufweisen.

6. Vorrichtung nach einem der Ansprüche 1 bis 5, **dadurch gekennzeichnet, dass** das Stechelement (14) in einer vorzugsweise durch einen zugeordneten Köcher (56) gebildeten Linearführung (26) gelagert ist.

7. Vorrichtung nach einem der Ansprüche 1 bis 6, **dadurch gekennzeichnet, dass** das Aufnahmeelement (16) in eine zu dem Sammelvolumen (44) hin führende Ausnehmung (42) des Stechelements (14) eingreift.

8. Vorrichtung nach einem der Ansprüche 1 bis 7, **dadurch gekennzeichnet, dass** das Stechelement (14) und das Aufnahmeelement (16) zusammengehörig als Einwegteil (76) ausgebildet sind.

9. Vorrichtung nach einem der Ansprüche 1 bis 8, **gekennzeichnet durch** ein zur Aufnahme einer Vielzahl von Stechelementen (14) und zugehörigen Aufnahmeelementen (16) ausgebildetes Magazin (54), insbesondere Scheiben-, Trommel- oder Stapelmagazin.

10. Vorrichtung nach einem der Ansprüche 1 bis 9, **dadurch gekennzeichnet, dass** das Aufnahmeelement (16) ein Messorgan für eine optische oder elektrochemische Messung in der-aufgenommenen Körperflüssigkeit (52) aufweist.

11. Vorrichtung nach Anspruch 10, **dadurch gekennzeichnet, dass** das Messorgan mit einem unter Flüssigkeitskontakt mit dem Inhaltsstoff der Körperflüssigkeit (52) reagierenden Testreagenz (46) beschichtet ist.

12. Vorrichtung nach einem der Ansprüche 1 bis 11, **dadurch gekennzeichnet, dass** das Aufnahmeelement (16) über einen in dem Stechelement (14) geführten Lichtleiter (38) mit einer Messeinheit (34) verbindbar oder verbunden ist.

13. Vorrichtung nach einem der Ansprüche 1 bis 12, **dadurch gekennzeichnet, dass** das Sammelvolumen (44) durch einen beidseitig offenen Schlitz oder eine einseitig offene Rinne des Stechelements (14) gebildet ist.

14. Vorrichtung zum Untersuchen von Körperflüssigkeiten, insbesondere nach einem der vorangehenden Ansprüche, mit einem längs einer Stechachse (48) in ein Körperteil (22) einstechbaren Stechelement (14), das ein Sammelvolumen (44) zum Sammeln von durch einen Einstich erhaltener Körperflüssigkeit (52) aufweist, und einem zum Nachweis eines Inhaltsstoffs der Körperflüssigkeit (52) ausgebildeten Aufnahmeelement (16), das mit Körperflüssigkeit (52) aus dem Sammelvolumen (44) beaufschlagbar ist, **dadurch gekennzeichnet, dass** das Sammelvolumen (44) weniger als 50 nl, bevorzugt weniger als 10 nl Körperflüssigkeit (52) aufnimmt.

15. Vorrichtung nach einem der Ansprüche 1 bis 14, **dadurch gekennzeichnet, dass** das Aufnahmeelement (16) an einer distalen Frontseite mit einem Aliquot der Körperflüssigkeit (52) aus dem Sammelvolumen (44) beaufschlagt ist, wobei das Aliquot weniger als 5 nl, bevorzugt weniger als 1 nl beträgt.

16. Stechgerät zum Einsatz mindestens einer als Einwegteil (76) ausgebildeten Vorrichtung nach einem der vorangehenden Ansprüche, mit einem durch einen Benutzer auslösbaren Stechantrieb (32), wobei das Einwegteil (76) in ungebrauchtem Zustand in einer ersten Position und nach dem Einstich in gebrauchtem Zustand in einer davon entfernten zweiten Position im Geräteinneren gelagert ist.

17. Stechgerät nach Anspruch 16 enthaltend eine mit dem Aufnahmeelement (16) zusammenwirkende Messeinheit (34) zum Nachweis des Inhaltsstoffs der Körperflüssigkeit (52).

18. Verfahren zum Untersuchen von Körperflüssigkeiten, insbesondere zur Durchführung von Blutzuckertests, bei welchem ein Stechelement (14) längs einer Stechachse (48) in ein Körperteil (22) eingestochen wird, wobei in einem Sammelvolumen (44) des Stechelements (14) durch den Einstich erhaltene Körperflüssigkeit (52) aufgenommen wird, und bei welchem einem zum Nachweis eines Inhaltsstoffs der Körperflüssigkeit (52) ein Aufnahmeelement (16) mit Körperflüssigkeit (52) aus dem Sammelvolumen (44) beaufschlagt wird, **dadurch gekennzeichnet, dass** während einer auf den Einstich folgenden Transferphase das Stechelement (14) und das Aufnahmeelement (16) in der Stechachse (48) relativ zueinander bewegt werden, so dass das Aufnahmeelement (16) in Stechrichtung in das Sammelvolumen (44) eintaucht.

19. Verfahren nach Anspruch 18, **dadurch gekennzeichnet, dass** der Abstand zwischen dem Aufnahmeelement (16) und einem distalen Stechorgan (40) des Stechelements (14) in der Transferphase verringert wird.

20. Verfahren nach Anspruch 18 oder 19, **dadurch gekennzeichnet, dass** das Aufnahmeelement (16) bei dem Einstich mit dem Stechelement (14) mitbewegt wird.

21. Verfahren nach Anspruch 18 oder 19, **dadurch gekennzeichnet, dass** das Stechelement (14) in einer hin- und hergehenden Stechbewegung angetrieben wird, während das Aufnahmeelement (16) ortsfest gehalten wird.

22. Verfahren nach einem der Ansprüche 18 bis 21, **dadurch gekennzeichnet, dass** das Stechelement (14) in der Transferphase außerhalb des die Körperflüssigkeit (52) enthaltenden Körperteils (22) befindlich ist.

23. Verfahren nach einem der Ansprüche 18 bis 22, **dadurch gekennzeichnet, dass** über das Aufnahmeelement (16) als Messorgan eine optische oder elektrochemische Messung in der aufgenommenen Körperflüssigkeit (52) durchgeführt wird.

24. Verfahren zur Sterilisierung einer als Einwegteil (76) ausgebildeten Vorrichtung nach einem der Ansprüche 1 bis 15, bei welchem das mit einem Testreagenz (46) beschichtete Aufnahmeelement (16) im Abstand von einem distalen Stechorgan (40) des Stechelements (14) gehalten wird und das Stechorgan (40) vorzugsweise mit einem Elektronenstrahl bestrahlt wird.

25. Verfahren nach Anspruch 24, **dadurch gekennzeichnet, dass** die Strahlung, insbesondere der Elektronenstrahl so auf das Stechorgan (40) fokussiert wird und/oder der Abstand zwischen Aufnahmeelement (16) und Stechorgan (40) so gewählt wird, dass das Testreagenz (46) durch die Strahlung nicht geschädigt wird.
